# EUROPÄISCHE PATENTSCHRIFT

(11) **EP 3 959 300 B1**
(45) Veröffentlichungstag und Bekanntmachung des Hinweises auf die Patenterteilung: **17.06.2026**
(21) Anmeldenummer: 20719614.8
(22) Anmeldetag: 15.04.2020
(51) Int. Cl.: C12M 1/00, C12M 1/12

(54) **VORRICHTUNG ZUR MECHANISCHEN STABILISIERUNG EINES ANSCHLUSSES, INSBESONDERE EINES SENSORANSCHLUSSES, AN EINEM FLEXIBLEN BEUTEL**
DEVICE FOR MECHANICALLY STABILIZING A CONNECTION, IN PARTICULAR A SENSOR CONNECTION, ON A FLEXIBLE BAG
DISPOSITIF DE STABILISATION MÉCANIQUE D'UNE CONNEXION, EN PARTICULIER D'UNE CONNEXION DE CAPTEURS, À UNE POCHE FLEXIBLE

(30) Priorität: 25.04.2019 DE 102019110742
(43) Veröffentlichungstag der Anmeldung: 02.03.2022
(73) Patentinhaber: Sartorius Stedim Biotech GmbH, 37079 Göttingen (DE)
(72) Erfinder: REGEN, Thomas, 37079 Goettingen (DE); SCHOLZ, Jochen, 37079 Goettingen (DE); DREHER, Thomas, 37079 Goettingen (DE); WOLLSCHMIDT, Regina, 37079 Goettingen (DE); GAERTNER, Sascha, 37079 Goettingen (DE); SCHUBERT, Jan-Eike, 37079 Goettingen (DE); HUSEMANN, Ute, 37079 Goettingen (DE); HILDEBRANDT, Sabrina, 37079 Goettingen (DE); SMANDZIK, Andrea, 37079 Goettingen (DE); ALTHUBER, Sharon-Angela, 37079 Goettingen (DE); PURMANN, Sebastian, 37073 Goettingen (DE)
(74) Vertreter: Novagraaf International SA
(86) Internationale Anmeldenummer: PCT/EP2020/060602
(87) Internationale Veröffentlichungsnummer: WO 2020/216660

(56) Entgegenhaltungen:
- EP-A1- 2 607 474
- EP-A1- 3 199 616
- US-A- 5 736 398
- US-A1- 2012 301 954
- US-A1- 2013 145 818
- US-A1- 2016 298 068
- US-A1- 2017 166 852

## Beschreibung

Die Erfindung betrifft eine Vorrichtung zur mechanischen Stabilisierung eines Anschlusses (Port), insbesondere eines Sensoranschlusses, an einem flexiblen Beutel (Bag), wie etwa einem Einweg-Bioreaktor.

Bei Einweg-Bioreaktoren oder anderen flexiblen Einweg-Beuteln, wie sie z. B. bei Einwegmischsystemen oder Einweglagersystemen im biopharmazeutischen Bereich eingesetzt werden, sind für die Datenaufzeichnung, Überwachung oder Regelung des darin stattfindenden Prozesses Sensoren essentiell. Dies können beispielsweise pH-, pO₂-Sonden oder Sensoren für die Bestimmung der Zellzahl sein. Oft sind die Sensoren - oder zumindest wesentliche Teile davon - als Einwegkomponenten mittels Kunststoffanschlüssen in die Beutelwand integriert, oder die Sensoren sind direkt an der Beutelwand angebracht, insbesondere durch Verschweißen oder Verkleben. Während sich der für die Messwerterfassung zuständige Teil eines solchen Sensors auf der dem Beutelinneren zugewandten Seite befindet, sind auf der nach außen weisenden Rückseite die für den Betrieb des Sensors erforderlichen Anschlüsse vorgesehen. Dabei kann es sich um elektrische, optische, Fluid- oder andere Anschlüsse handeln.

Üblicherweise wird ein flexibler Einweg-Beutel vor Beginn eines Prozesses in eine starren Behälter, typischerweise aus Edelstahl, gelegt, sodass sich der Beutel während und nach dem Befüllen mit einem Medium aufgrund des hydrostatischen Drucks an die Innenwand des äußeren Behälters legt und von dieser gestützt wird. Der äußere Behälter weist an der Stelle, die dem in die Beutelwand integrierten Sensor gegenüberliegt, eine Fensteröffnung auf, damit der Sensor, insbesondere dessen rückseitige Anschlüsse, von außen zugänglich ist. Im Bereich der Fensteröffnung kann der äußere Behälter aber keine Stützfunktion für den Beutel ausüben. Die Beutelwand ist deshalb in dem Bereich um den integrierten Sensor besonders gefährdet, da der gefüllte Beutel teilweise aus der Fensteröffnung gedrückt wird und dadurch einer besonders hohen Belastung ausgesetzt ist. Es gilt jedoch unbedingt zu vermeiden, dass der Beutel aufreißt und Medium unkontrolliert aus dem Beutel austritt.

Die US 2012/301954 A1 befasst sich mit der Befestigung eines Sensors an einem Beutel und geht davon aus, dass der Sensor nicht dazu geeignet ist, am Bag festgeschweißt zu werden, weshalb vorgeschlagen wird, ein Halteteil am Bag festzuschweißen.

Die US 2013/145818 A1 betrifft eine Einweg-Sensor-Einheit zum Anbringen eines Sensors an einen flexiblen Einweg-Beutelreaktor. Die Sensorhalterung ist als Flansch ausgebildet und mit dem flexiblen Beutel verbunden. Das Anschlussteil wird über einen Klemmmechanismus befestigt und mittels einer Überwurfkappe gehalten.

Aufgabe der Erfindung ist es, mittels einer kostengünstigen und einfach handzuhabenden Vorrichtung eine Stabilisierung eines flexiblen Beutels im Bereich eines in die Beutelwand integrierten Sensors zu ermöglichen.

Die Aufgabe der Erfindung wird gelöst durch eine Vorrichtung zur mechanischen Stabilisierung eines Anschlusses, insbesondere eines Sensoranschlusses, an einem flexiblen Beutel mit den Merkmalen des Anspruchs 1. Vorteilhafte und zweckmäßige Ausgestaltungen der erfindungsgemäßen Stabilisierungsvorrichtung sind in den zugehörigen Unteransprüchen angegeben.

Die erfindungsgemäße Vorrichtung dient zur mechanischen Stabilisierung eines Anschlusses, insbesondere eines Sensoranschlusses, an einem flexiblen Beutel und umfasst ein starres Stützteil, das aus wenigstens zwei Teilen, vorzugsweise Streben, zusammensetzbar ist. Die Teile umgeben im zusammengesetzten Zustand zusammen einen freien Bereich oder eine Öffnung, in die ein in den Beutel integriertes starres Anschlussteil, insbesondere ein Sensor platziert werden kann. Der freie Bereich oder die Öffnung weist im zusammengesetzten Zustand der Teile einen kleineren Durchmesser auf als das Anschlussteil, sodass die Teile eine Lastaufnahme für das Anschlussteil bilden.

Zumindest im Zusammenhang mit dem erfindungsgemäßen Stützteil und das Anschlussteil ist unter "starr" ein - im Gegensatz zum flexiblen Beutel - im Wesentlichen festes, knick- und bruchfestes Material zu verstehen, das aber nicht zwingend vollkommen unverformbar sein muss, sondern eine gewisse Biegsamkeit aufweisen darf.

Die Erfindung beruht auf dem Grundgedanken, dass der gefährdete Bereich des flexiblen Beutels mit dem Anschluss, der aus der Fensteröffnung des umgebenden starren Behälters gedrückt werden könnte, durch eine starre Platte stabilisiert werden kann. Das Stützteil der erfindungsgemäßen Vorrichtung ermöglicht genau dies, indem es sich zumindest an gegenüberliegenden Rändern der Fensteröffnung abstützt und dadurch den gefährdeten Bereich des Beutels entlastet. Dabei wird das Stützteil durch das im flexiblen Beutel befindliche und aufgrund der Schwerkraft und der räumlichen Begrenzungen nach außen drängende Medium automatisch gegen die Ränder der Fensteröffnung gedrückt. Zusätzliche Maßnahmen zur Fixierung des Stützteils an der Fensteröffnung sind also nicht erforderlich.

Die erfindungsgemäße Stabilisierungsvorrichtung zeichnet sich durch ihren einfachen Aufbau, der ohne separates Befestigungselement auskommt, und ihre einfache Handhabung aus. Ansonsten bietet sie im Wesentlichen die gleichen Vorteile wie die Vorrichtung gemäß dem ersten Aspekt.

Bei einer bevorzugten Ausführungsform der erfindungsgemäßen Vorrichtung ergeben die Teile im zusammengesetzten Zustand ein im Wesentlichen U-förmiges Stützteil. Die Längsschenkel und ggf. der die Schenkel verbindende Abschnitt des Stützteils können leicht hinter das Anschlussteil, d. h. im bevorzugten Anwendungsfall den Sensor, geschoben werden, um so die Lastaufnahme zu bilden.

Um das Zusammensetzen der Teile besonders einfach und sicher zu gestalten, können die Teile mithilfe eines Verbindungselements, beispielsweise eines Stifts oder dergleichen, verbunden werden. Ein Abschnitt des Verbindungselements steht von einem der Teile, aus dem das Stützteil gebildet ist, vor und ist in eine an einem anderen Teil des Stützteils vorgesehene, auf den vorstehenden Abschnitt des Verbindungselements abgestimmte Öffnung einführbar.

Gemäß einer bevorzugten Gestaltung des Stützteils weist eine Unterseite der Teile, aus denen das Stützteil gebildet ist, eine auf die Form des Anschlussteils abgestimmte Aufnahme auf. Dadurch ergibt sich für den Benutzer eine definierte Positionierung der Teile, wenn er diese zusammenschiebt, und das Anschlussteil (Sensor) kann nicht ohne Weiteres vom Stützteil abrutschen.

Gemäß einer weiteren vorteilhaften Gestaltung weist das Stützteil an entgegengesetzten Enden abgeflachte Vorsprünge auf. Die abgeflachten Vorsprünge sind dazu vorgesehen, gegenüberliegende Randabschnitte einer Fensteröffnung in einem starren Behälter zu hintergreifen, in dem der Beutel während eines Prozesses aufgenommen ist. Die Abstände der entgegengesetzten Vorsprünge sind auf die entsprechende Abmessung der Fensteröffnung abgestimmt, sodass sich eine in dieser Hinsicht für den Benutzer eindeutige Positionierung des Stützteils bzw. dessen Teilen ergibt. Falls das Stützteil aus mehreren Teilen zusammengesetzt ist, können diese nach ihrer durch die Vorsprünge vorgegebenen Anordnung noch in einer Querrichtung zusammengeschoben werden, um die endgültige Lastaufnahme für das Anschlussteil (Sensor) zu bilden.

Besonders vorteilhaft ist eine Ausführungsform der erfindungsgemäßen Vorrichtung, bei der das Stützteil als Einweg-Komponenten ausgelegt ist und aus einem sterilisierbaren Kunststoff besteht. Somit kann die erfindungsgemäße Vorrichtung bereits vorab an einem Einweg-Beutel installiert werden, und der gesamte Verbund kann vor seiner Auslieferung in einem Schritt gleichzeitig sterilisiert werden, insbesondere durch Gammastrahlung.

Das Stützteil kann aber auch als Mehrweg-Komponente ausgelegt sein. In diesem Fall ist es vorzugsweise mehrfach zusammensetz- und zerlegbar, sodass es mehrmals an einem Sensor oder einem anderen Anschlussteil montiert und demontiert werden kann.

Die Erfindung schafft auch eine Vorrichtungsanordnung mit einem flexiblen Beutel, einem in eine Wand des Beutels integrierten starren Anschlussteil und einer Stabilisierungsvorrichtung, wie sie oben gemäß dem ersten Aspekt oder dem zweiten Aspekt definiert wurde. Die gesamte Vorrichtungsanordnung kann als Einheit verpackt, gelagert und ausgeliefert werden. Fehler seitens des Anwenders beim Aufbau vor der Inbetriebnahme des Beutels sind dadurch ausgeschlossen.

In der bevorzugten Anwendung der Erfindung ist der Beutel in einem starren Behälter aufgenommen und wird von diesem abgestützt. Der starre Behälter hat eine Fensteröffnung, durch die das in den Beutel integrierte Anschlussteil zugänglich ist.

Um einen optimalen Halt des Stützteils für den Fall zu gewährleisten, dass die Innenwand des Behälters im Bereich der Fensteröffnung keine glatte oder gleichmäßige Kontur hat, weist das Stützteil vorzugsweise eine Form auf, die an diese Kontur angepasst ist.

Gemäß der bevorzugten Anwendung der Erfindung ist der flexible Beutel ein Einweg-Bioreaktor, und das Anschlussteil ist ein Sensor.

Für den Anwender ist es besonders vorteilhaft, wenn er bei einem Prozessaufbau möglichst wenige Reinigungsarbeiten durchführen und möglichst wenige Komponenten sterilisieren muss. Deshalb sieht die Erfindung in einer bevorzugten Ausführungsform vor, dass der Beutel, das Anschlussteil und die Stabilisierungsvorrichtung als sterilisierbare Einwegkomponenten ausgelegt sind. Somit kann die gesamte erfindungsgemäße Vorrichtungsanordnung (flexibler Beutel, in die Beutelwand integriertes Anschlussteil und Stabilisierungsvorrichtung mit Stützteil und Befestigungselement) als vormontierte und vorsterilisierte Einheit an den Anwender geliefert werden.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figur 1 eine Draufsicht auf die Einzelteile einer nicht erfindungsgemäßen Vorrichtung zur mechanischen Stabilisierung eines Sensoranschlusses nach einer ersten Ausführungsform;
- Figur 2 eine Draufsicht auf das Stützteil der Vorrichtung aus Figur 1, das auf einen in einen Beutel integrierten Sensor aufgelegt ist;
- Figur 3 eine Draufsicht auf die Vorrichtung aus Figur 1 im transportfähigen Zustand;
- Figur 4 eine Schnittansicht der Vorrichtung aus Figur 1 im transportfähigen Zustand;
- Figur 5 eine perspektivische Ansicht einer nicht erfindungsgemäßen Vorrichtung nach einer zweiten Ausführungsform im transportfähigen Zustand;
- Figur 6 einen Ausschnitt eines starren Behälters mit einem darin aufgenommenen flexiblen Bioreaktor mit der Vorrichtung aus Figur 1 ohne Befestigungselement;
- Figur 7 eine perspektivische Ansicht der Einzelteile einer nicht erfindungsgemäßen Vorrichtung nach einer dritten Ausführungsform;
- Figur 8 eine perspektivische Detailansicht der Vorrichtung aus Figur 7 in einer ersten Drehstellung des Befestigungselements;
- Figur 9 eine perspektivische Detailansicht der Vorrichtung aus Figur 7 in einer zweiten Drehstellung des Befestigungselements;
- Figur 10 eine perspektivische Ansicht der Oberseite eines Stützteils einer erfindungsgemäßen Vorrichtung nach einer vierten Ausführungsform;
- Figur 11 eine seitliche Schnittansicht des Stützteils aus Figur 10;
- Figur 12 eine Ansicht der Unterseite des Stützteils aus Figur 10;
- Figur 13 eine perspektivische Ansicht der gesamten erfindungsgemäßen Vorrichtung nach der vierten Ausführungsform und
- Figuren 14 bis 17 aufeinanderfolgende Schritte für die Installation der Vorrichtung nach der vierten Ausführungsform.

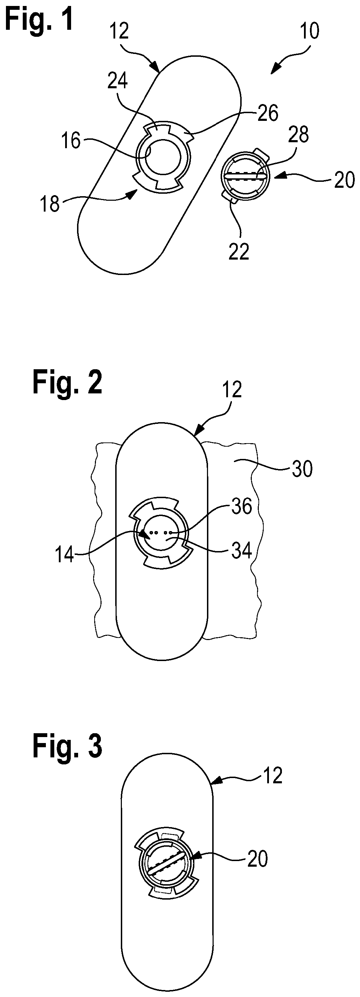

In den Figuren 1 bis 4 ist eine beispielhafte erste Ausführungsform einer Vorrichtung gezeigt 10, die zur Stabilisierung eines Anschlusses (Port), hier eines Sensoranschlusses (Sensorport), vorgesehen ist, der in die Wand eines flexiblen Beutels (Bag) integriert ist, insbesondere eines Einweg-Bioreaktors.

Die Stabilisierungsvorrichtung 10 umfasst ein starres, knick- und bruchfestes Stützteil 12 in Form einer vergleichsweise dünnen Platte mit einer Aufnahme für einen zur Verwendung im Bioreaktor vorgesehenen Sensor 14 (in Figur 1 nicht gezeigt). Das Stützteil 12 besteht aus einem stabilen Kunststoff oder einem anderen geeigneten sterilisierbaren Material.

Die Aufnahme für den Sensor 14 ist im Wesentlichen durch eine Öffnung 16 im Stützteil 12 gebildet. Im dargestellten Ausführungsbeispiel ist die Öffnung 16 kreisrund und hat einen etwas größeren Durchmesser als der ebenfalls kreisrunde, insgesamt im Wesentlichen scheibenförmige Sensor 14. Allgemein sind Form und Abmessungen des Sensors 14 und der Öffnung 16 so aufeinander abgestimmt, dass der Sensor 14 in der Öffnung 16 gar kein oder kein großes Spiel hat (siehe Figur 2).

Auf einer ersten Seite des plattenförmigen Stützteils 12 ist die Öffnung 16 von einer vorstehenden Halteeinrichtung 18 umgeben, die hier einstückig mit dem Stützteil 12 ausgeführt ist. Die Halteeinrichtung 18 dient zur Aufnahme und zum Halten eines Befestigungselements 20, das hier als (Schraub-)Kappe ausgeführt ist. Der Durchmesser des Befestigungselements 20 ist größer als der der Öffnung 16 im Stützteil 12. Das Befestigungselement 20 besteht ebenfalls aus einem stabilen Kunststoff oder einem anderen geeigneten sterilisierbaren Material.

Zur Aufnahme und zum Halten des Befestigungselements 20 am Stützteil 12 können dessen Halteeinrichtung 18 und das Befestigungselement 20 aufeinander abgestimmte Gewinde aufweisen, sodass das Befestigungselement 20 am Stützteil 12 festgeschraubt werden kann.

Bei den in den Figuren 1 bis 6 dargestellten Ausführungsbeispielen ist jedoch ein Drehverschluss vorgesehen, bei dem in einer oder mehreren definierten Drehstellungen des Befestigungselements 20 radial vom Befestigungselement 20 vorstehende Abschnitte 22 in passende, nach oben offene Aufnahmebereiche 24 der Halteeinrichtung 18 gelegt und anschließend durch Drehung des Befestigungselements 20 um einen bestimmten Winkel in überdeckte Aufnahmebereiche 26 geklemmt werden können. Zusätzlich kann ein Anschlag vorgesehen sein, der eine haptische Rückmeldung an den Anwender gibt und ein Überdrehen verhindert.

Dieses Befestigungskonzept kann in einer erweiterten Ausführung auch mit rampenförmigen Abschnitten am Befestigungselement 20 und/oder an den Aufnahmebereichen 24 nach Art eines Bajonett-Verschlusses gestaltet sein.

Das Befestigungselement 20 hat auf seiner Innenseite ein Innengewinde, das auf ein Außengewinde des Sensors 14 abgestimmt ist, wie später noch genauer erläutert wird.

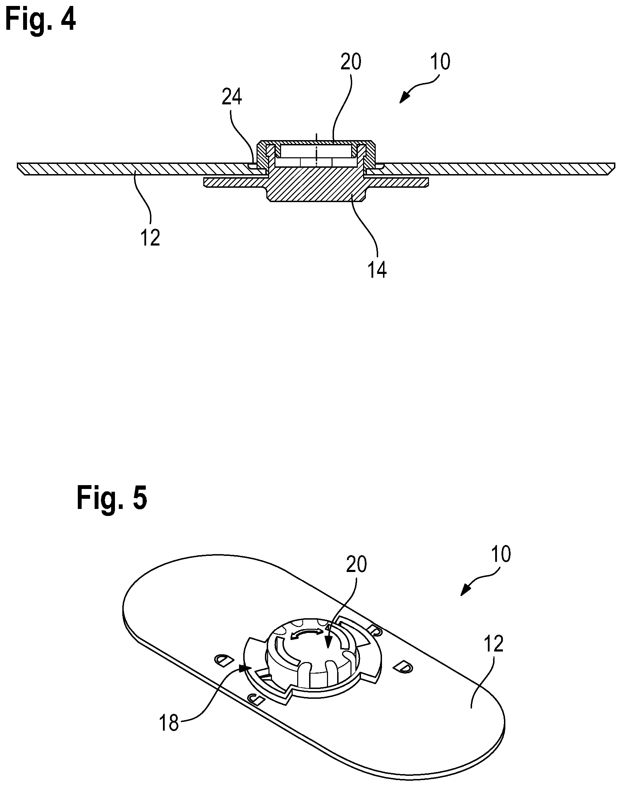

Zur leichteren Handhabung hat das Befestigungselement 20 einen abstehenden Greifabschnitt 28 (Figur 1) oder eine profilierte Oberfläche (Figur 5), sodass ein Benutzer das Befestigungselement 20 leicht greifen und in der Halteeinrichtung 18 drehen kann.

Nachfolgend werden die Handhabung und die Funktion der Stabilisierungsvorrichtung 10 anhand eines konkreten Beispiels mit einem Bioreaktor als Beutel und einem Sensor 14 als Anschlussteil beschrieben.

Voraussetzung für die Verwendung der Stabilisierungsvorrichtung 10 ist in diesem Fall ein bereits in einen flexiblen Einweg-Beutel 30 integrierter, insbesondere eingeschweißter Sensor 14. Der Sensor 14 ist so in eine Wand des Beutels 30 eingearbeitet, dass seine Vorderseite mit dem messempfindlichen Teil, der mit dem Medium im Beutel 30 in Berührung kommen oder diesem zumindest zugewandt sein soll, dem Beutelinneren zugewandt ist, während seine Rückseite 34 mit elektrischen, optischen oder anderen erforderlichen Anschlüssen 36 nach außen weist. Selbstverständlich ist sicherzustellen, dass der Beutel 30 im Bereich des Sensors 14 vollkommen dicht ist.

Wie in Figur 2 gezeigt, wird das Stützteil 12 so auf die Außenwand des Beutels 30 gelegt, dass die der Oberseite mit der Halteeinrichtung 18 gegenüberliegende Unterseite des Stützteils 12 der Beutelaußenwand zugewandt ist und der Sensor 14 mit seiner nach oben weisenden Rückseite 34 in die Öffnung 16 des Stützteils 12 ragt. Das Stützteil 12 sollte dabei, sofern dies für den späteren Gebrauch von Bedeutung ist, in die gewünschten Orientierung (Drehstellung) relativ zur Beutelwand gebracht werden. Danach wird das Befestigungselement 20 je nach Befestigungskonzept durch Schrauben, Drehen, Klemmen oder dergl. an der Halteeinrichtung 18 des Stützteils 12 angebracht.

Bei den in den Figuren 1 bis 6 gezeigten Ausführungsbeispielen wird durch das Drehen des Befestigungselements 20 in der Halteeinrichtung 18 gleichzeitig eine Verbindung zwischen dem starren Sensor 14 und dem Befestigungselement 20 hergestellt. Diese Verbindung wird durch die aufeinander abgestimmten Gewinde des Befestigungselements 20 und des Sensors 14 erreicht. Genauer gesagt greift durch das Drehen des Befestigungselements 20 dessen Innengewinde in das Außengewinde des Sensors 14, sodass nach dem Anbringen des Befestigungselements 20 der Beutel 30 mit dem Sensor 14 und das Stützteil 12 mit dem Befestigungselement 20 einen Verbund bilden, wobei das Stützteil 12 in der gewünschten Orientierung relativ zur Beutelwand gehalten wird (siehe Figur 4).

Es ist auch eine Ausbildung möglich, bei der nach dem Auflegen des Stützteils 12 auf den Beutel 30 das Befestigungselement 20 zunächst durch mehrere Umdrehungen auf den Sensor 14 aufgeschraubt wird. Das Befestigungselement 20 wird so weit aufgeschraubt, bis die radial vom Befestigungselement 20 vorstehenden Abschnitte 22 in die passenden Aufnahmebereiche 24 der Halteeinrichtung 18 gelangen und dann durch ein abschließendes Drehen des Befestigungselements 20 um einen bestimmten Winkel in die überdeckten Aufnahmebereiche 26 geklemmt werden.

Das Außengewinde muss nicht zwingend am Sensor 14 selbst vorgesehen sein, sondern kann alternativ an einer fest mit dem Sensor 14 und/oder dem Beutel 30 verbundenen Halterung gebildet sein.

Die Stabilisierungsvorrichtung 10 einschließlich des Stützteils 12 und des Befestigungselements 20 kann vor ihrem bestimmungsgemäßen Gebrauch vorsterilisiert und im sterilisierten Zustand verpackt und an einen Anwender geliefert werden. Typischerweise wird die Stabilisierungsvorrichtung 10 vorab an dem in den flexiblen Einweg-Beutel 30 integrierten Sensor 14 angebracht und der Beutel 30 zusammen mit der Stabilisierungsvorrichtung 10 verpackt und ausgeliefert. Da auch der Sensor 14 als Einweg-Komponente ausgelegt ist, kann die gesamte Einheit (Beutel 30 mit Sensor 14 und Stabilisierungsvorrichtung 10 mit Stützteil 12 und Befestigungselement 20) vor dem Verpacken oder kurz vor dem Gebrauch beim Anwender sterilisiert werden, insbesondere durch Gammastrahlung.

Bei der in Figur 5 gezeigten zweiten Ausführungsform ist das Stützteil 12 der Stabilisierungsvorrichtung 10 im Einsatz zu sehen. Ein als flexibler Kunststoff-Beutel 30 ausgeführter und mit einem Medium gefüllter Einweg-Bioreaktor ist in einem starren wiederverwendbaren Behälter 38 aus Edelstahl oder einem anderen geeigneten Material aufgenommen und wird von diesem gehalten und abgestützt. Der äußere Behälter 38 hat eine Fensteröffnung 40, durch die ein bestimmter Abschnitt der Außenwand des Bioreaktors sichtbar und zugänglich ist. Genau in diesem Bereich befindet sich der in die Bioreaktorwand integrierte Sensor 14 mit der Stabilisierungsvorrichtung 10. Form und Abmessungen der Fensteröffnung 40 und des Stützteils 12 sind so aufeinander abgestimmt, dass sich das Stützteil 12 an seinen Außenbereichen mit einer ausreichend großen Stützfläche an der die Fensteröffnung 40 umgebenden Innenwand des äußeren Behälters 38 abstützen kann. Material und Stärke (Dicke) des Stützteils 12 sind so gewählt, dass sich das Stützteil 12 durch den Druck des Mediums im flexiblen Einweg-Bioreaktor im Bereich der Fensteröffnung 40 nicht oder nur unwesentlich verbiegt, auf keinen Fall jedoch knickt oder bricht. Die Stabilisierungsvorrichtung 10, genauer gesagt das Stützteil 12, sorgt somit dafür, dass die flexible Wand des Bioreaktors im Bereich des Sensors 14 entlastet wird, sodass ein Durchteten der Beutelwand durch das Fenster 40 oder eine Beschädigung des Bioreaktors in diesem gefährdeten Bereich vermieden wird.

Der Sensor 14 kann in Betrieb genommen werden, indem das Befestigungselement 20 abgenommen und die erforderlichen Anschlüsse, z. B. für die Messelektronik, hergestellt werden. Dies ist möglich, da der Sensorbereich des Bioreaktors durch die Fensteröffnung 40 im äußeren Behälter 38 zugänglich ist. Das Medium im Bioreaktor drückt dessen Wand gegen die Innenwand des äußeren Behälters 38, sodass das Stützteil 12 in der gewünschten Position an der Fensteröffnung 40 verbleibt und nicht verrutscht und sich auch nicht vom Sensor 14 löst.

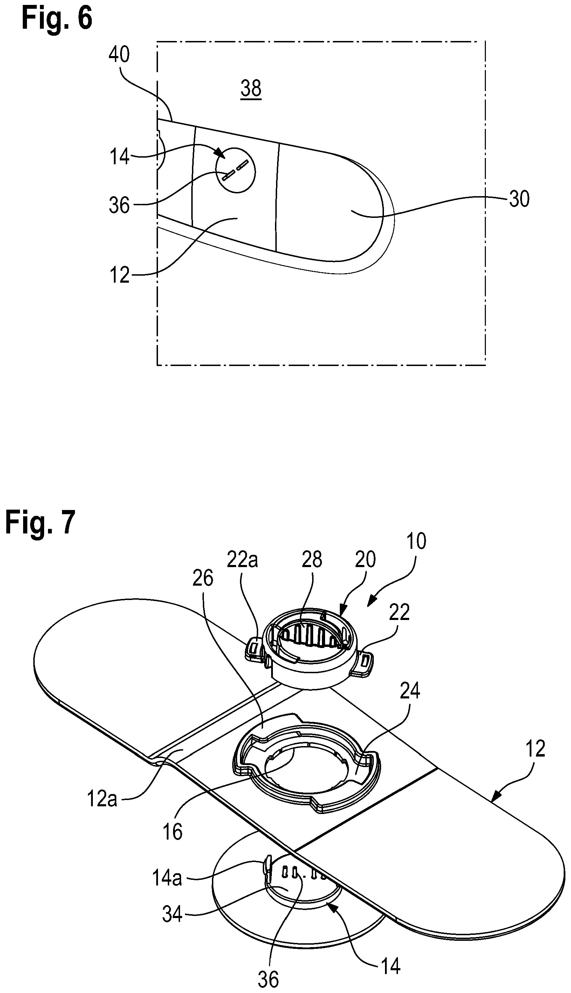

Die Figuren 7 bis 9 zeigen eine etwas anders gestaltete dritte Ausführungsform der Stabilisierungsvorrichtung 10. Diese Ausführungsform unterscheidet sich in der Form des Stützteils 12 und in der Art der Verriegelung des Befestigungselements 20.

Das Stützteil 12 ist auch hier im Wesentlichen plattenförmig, jedoch nicht vollständig eben. Vielmehr weist das Stützteil 12 eine gebogene Form auf, die an eine entsprechende Kontur der Innenwand des äußeren Behälters 38 im Bereich der Fensteröffnung 40 angepasst ist. Wie in Figur 7 zu sehen ist, weist das Stützteil 12 einen bogenförmigen Abschnitt 12a auf. Selbstverständlich sind auch andere Formgebungen möglich, um etwaige Unebenheiten der Behälterinnenwand im Bereich der Fensteröffnung 40 auszugleichen. Auch eine insgesamt gekrümmte Form ist möglich.

Des Weiteren ist eine besondere Bajonettverriegelung für das Befestigungselement 20 vorgesehen. Wie zuvor beschrieben werden in einer definierten ersten Drehstellung des Befestigungselements 20 die radial vorstehenden Abschnitte 22 in passende, nach oben offene Aufnahmebereiche 24 der Halteeinrichtung 18 gelegt und anschließend durch Drehung des Befestigungselements 20 um einen bestimmten Winkel in eine zweite Drehstellung in überdeckte Aufnahmebereiche 26 geklemmt. Die Klemmwirkung zwischen dem Befestigungselement 20 und der Halteeinrichtung 18 des Stützteils 12 wird hier durch rampenförmige Abschnitte 22a auf den vorstehenden Abschnitten 22 erreicht. Zur leichteren Handhabung hat das Befestigungselement 20 hier einen abstehenden Greifabschnitt 28, der zusätzlich noch eine profilierte Oberfläche aufweist.

Das Befestigungselement 20 hat auf seiner Innenseite jedoch kein Innengewinde, und auch der Sensor 14 hat kein Außengewinde. Stattdessen weist der Sensor 14 auf seiner von vom Beutelinneren abgewandten Rückseite 34 mehrere (hier zwei gegenüberliegende) vorstehende Klemmabschnitte 14a, 14b auf, die elastisch auslenkbar sind. Diese Klemmabschnitte 14a, 14b sind auf Klemmbereiche 20a, 20b des Befestigungselements 20 abgestimmt, sodass in der zweiten Drehstellung des Befestigungselements 20 auch eine Klemmverbindung zwischen dem Befestigungselement 20 und dem Sensor 14 hergestellt wird.

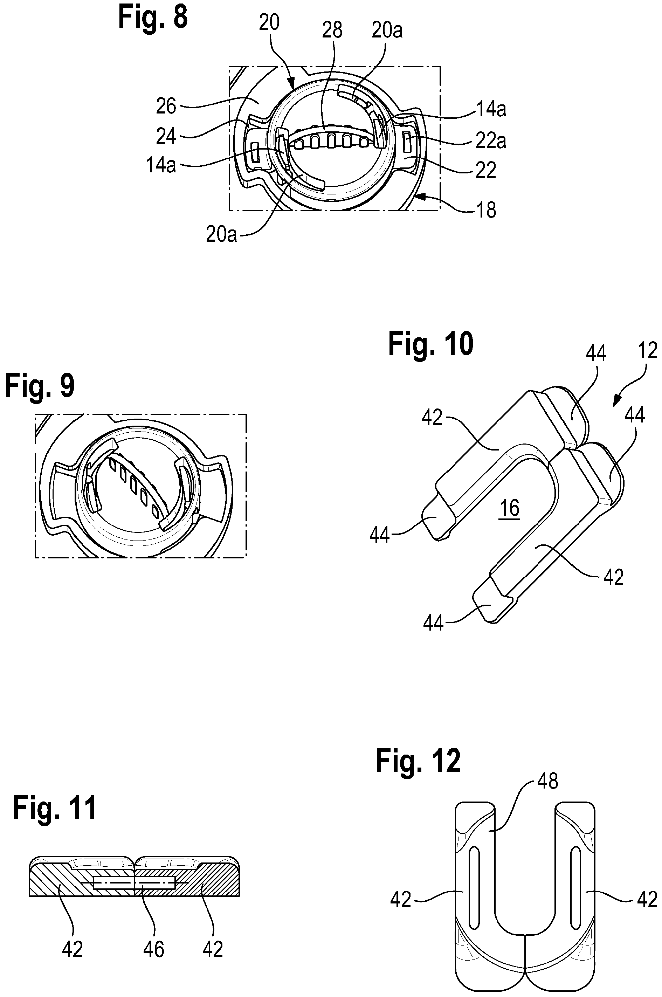

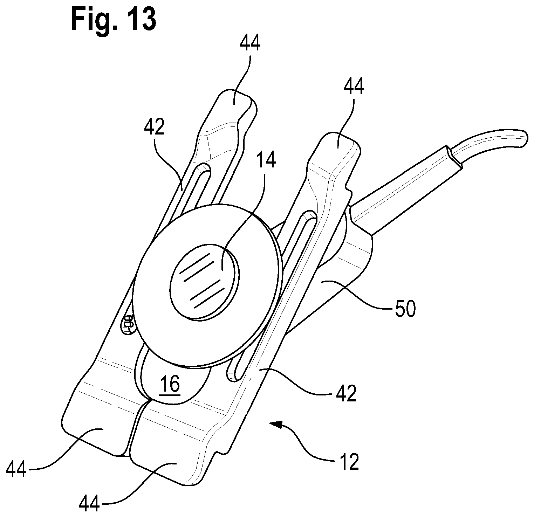

In den Figuren 10 bis 12 ist eine vierte Ausführungsform des Stützteils 12 gezeigt, das für eine mehrfache Verwendung vorgesehen ist, d. h. dieses Stützteil 12 ist nicht zwingend als Einweg-Komponente ausgelegt. Diese Ausführungsform, die in Figur 13 als Ganzes im fertig montierten Zustand mit einem Sensor 14 und einem Anschlusselement 50 gezeigt ist, kommt ohne separates Befestigungselement aus.

Bei der vierten Ausführungsform besteht das Stützteil 12 aus wenigstens zwei Einzelteilen, hier insbesondere in Form von zwei Streben 42, die zusammengesetzt im Wesentlichen eine U-förmige Gestalt ergeben. Die Teile können zusammengesetzt auch die Form eines Rings oder dergleichen ergeben, solange in der Mitte ein freier Bereich oder eine Öffnung 16 mit einem Durchmesser entsteht, der kleiner als der Durchmesser des Sensors 14 bzw. dessen Halterung ist.

Die lastaufnehmenden Streben 42 können aus robusten Kunststoffen, z. B. PEEK (Polyetheretherketon), aber auch aus Metall gefertigt sein.

In Figur 10 ist zu sehen, dass die Streben 42 an ihren Längsenden auf der Oberseite jeweils eine Stufe aufweisen, d. h. die Streben 42 haben an ihren Längsenden abgeflachte Vorsprünge 44. Die Längserstreckung der Streben 42 zwischen den Stufen, d. h. ohne die Vorsprünge 44, entspricht einer Abmessung der Fensteröffnung 40 im Behälter 38, vorzugsweise der vertikalen Abmessung der Fensteröffnung 40.

Wie in Figur 11 zu erkennen ist, sind die beiden Streben 42 durch ein Verbindungselement 46, wie etwa einen Stift oder eine Leitschiene, miteinander verbunden. Das Verbindungselement 46 ist an einer der beiden Streben 42 befestigt und steht von dieser hervor. Die andere Strebe 42 weist eine auf das Verbindungselement 46 abgestimmte Öffnung auf, sodass bei einem Zusammenstecken der beiden Streben 42 der vorstehende Abschnitt des Verbindungselements 46 in die Öffnung eingeführt wird und dadurch sichergestellt ist, dass die Streben 42 die in den Figuren 10 bis 12 gezeigte korrekte Montageposition einnehmen.

Die in Figur 12 gezeigte Unterseite der Streben 42 weist vertiefte bzw. ausgesparte Bereiche auf, die so geformt sind, dass sie im zusammengesteckten Zustand der Streben 42 (Montageposition) eine auf die Form des Sensors 14 bzw. dessen Halterung abgestimmte (hier im Wesentlichen kreisrunde) Aufnahme 48 bilden.

Im Unterschied zu einer einteiligen Ausführung ist ein mehrfaches Anbringen und Abnehmen des Stützteils 12 an bzw. von einem installierten Sensor 14 bzw. dessen Halterung am Beutel 14 möglich, da die Streben 14 mehrfach zusammengesetzt und auseinandergenommen werden können.

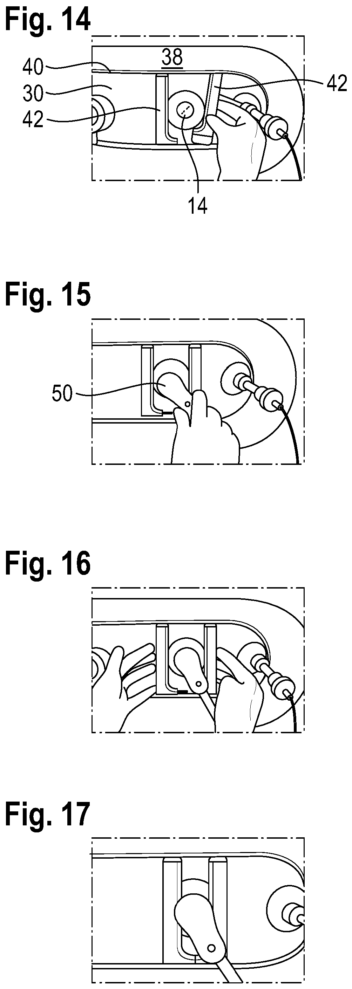

Nachfolgend wird anhand der Figuren 14 bis 17 die Installation des Stützteils 12 nach der vierten Ausführungsform beispielhaft beschrieben.

Der als flexibler Kunststoff-Beutel 30 ausgeführte und in dem starren, wiederverwendbaren Behälter 38 aus Edelstahl oder einem anderen geeigneten Material aufgenommene Einweg-Bioreaktor wird bis zu einem vorgegebenen minimalen Volumen mit dem für den durchzuführenden Prozess (z. B. Zellkultivierung) vorgesehenen Medium befüllt. Der Bioreaktor wird so platziert, dass sich der in die Bioreaktorwand integrierte Sensor 14 im Bereich der Fensteröffnung 40 des Behälters 38 befindet.

Wie in Figur 14 gezeigt, werden die beiden Streben 42 einzeln zwischen den Rand der Fensteröffnung 40 und die Außenwand des Bioreaktors geklemmt. Die Streben 42 werden dabei auf entgegengesetzten Seiten des Sensors 14 positioniert (hier links und rechts vom Sensor 14). Die Oberseite der Streben 42 weist nach außen, d. h. die mit Ausnahme der vertieften Bereiche ebene und glatte Unterseite der Streben 42 liegt an der Außenwand des Bioreaktors an, sodass der Bioreaktor nicht beschädigt werden kann. Die Vorsprünge 44 der Streben 42 hintergreifen den Rand der Fensteröffnung 40, während die dickeren Mittelabschnitte der Streben 42 im Bereich der Fensteröffnung 40 für den Benutzer gut greifbar sind.

Anschließend werden die erforderlichen Anschlüsse, z. B. für die Messelektronik, hergestellt (siehe Figur 15). Im dargestellten Ausführungsbeispiel wird hierzu ein Anschlusselement 50 in Form eines Vorverstärkers (pre-amplifier) auf die Rückseite des Sensors 14 aufgesteckt oder auf andere Weise angebracht.

Danach wird das Stützteil 12 in seine Montageposition überführt, indem, wie in Figur 16 gezeigt, die beiden Streben 42 zusammengeschoben werden, bis der überstehende Teil des Verbindungselements 44 der einen Strebe 42 vollständig in die zugehörige Öffnung der anderen Strebe 42 eingeführt ist. Die vertieften bzw. ausgesparten Bereiche auf der Unterseite der Streben 42 erleichtern es dem Benutzer, die Streben 42 über den Rand des Sensors 14 bzw. dessen Halterung zu schieben. Nach dem Zusammenschieben befindet sich der Sensor 14 bzw. dessen Halterung vollständig in der Aufnahme 48.

Der endgültige Montagezustand ist in Figur 17 zu sehen. Der Bioreaktor kann nun weiter bis zum gewünschten Arbeitsvolumen mit Medium befüllt werden. Das zusätzliche Medium drückt den Sensor 14 bzw. dessen Halterung noch fester gegen das Stützteil 12, das einen großen Anteil der Last aufnimmt und auf den Behälter 38 überträgt. Dank des an den Rand der Fensteröffnung 40 gedrückten Stützteils 12, und insbesondere dessen Aufnahme 38, ist der Sensor 14 fixiert und kann nicht verrutschen.

Gegebenenfalls kann das Anschlusselement 50 auch erst nach der vollständigen Montage des Stützteils 12 am Sensor 14 angebracht werden.

Die Demontage des Stützelements 12 erfolgt im Wesentlichen in umgekehrter Reihenfolge der oben beschriebenen Installationsschritte.

Stützteile 12 können in mehreren Größen bzw. Formen bereitgestellt werden, insbesondere angepasst an standardisierte bzw. typische Größen und Formen von Sensoren 14 und Fensteröffnungen 40.

Selbstverständlich können bestimmte Merkmale der oben beschriebenen Ausführungsformen in geeigneter Weise miteinander kombiniert werden.

Die Stabilisierungsvorrichtung 10 eignet sich nicht nur für Bioreaktoren, sondern grundsätzlich für alle flexiblen Einweg-Beutel mit integrierten Sensoren 14, wie sie z. B. auch bei Einweg-Mischsystemen oder Einweg-Lagersystemen eingesetzt werden. Außerdem ist die Stabilisierungsvorrichtung 10 nicht nur für Sensoranschlüsse, sondern auch für andere Anschlüsse, z. B. Schlauch- oder Konnektoranschlüsse geeignet, die in einen flexiblen Beutel 30 integriert sind. Die Aufnahme des Stützteils 12 ist dann nicht auf Form und Abmessungen eines Sensors 14, sondern eines anderen starren Anschlussteils abgestimmt.

### Bezugszeichenliste

- 10: Stabilisierungsvorrichtung
- 12: Stützteil
- 12a: bogenförmiger Abschnitt des Stützteils
- 14: Sensor
- 14a: Klemmabschnitte des Sensors
- 16: Öffnung im Stützteil
- 18: Halteeinrichtung des Stützteils
- 20: Befestigungselement
- 20a: Klemmbereiche des Befestigungselements
- 22: vorstehende Abschnitte des Befestigungselements
- 22a: rampenförmige Abschnitte auf den vorstehenden Abschnitten
- 24: offene Aufnahmebereiche der Halteeinrichtung
- 26: überdeckte Aufnahmebereiche der Halteeinrichtung
- 28: Greifabschnitt des Befestigungselements
- 30: Beutel
- 34: Rückseite des Sensors
- 36: Anschlüsse des Sensors
- 38: Behälter
- 40: Fensteröffnung des Behälters
- 42: Streben
- 44: Vorsprünge
- 46: Verbindungselement
- 48: Aufnahme
- 50: Anschlusselement

## Patentansprüche

1. Vorrichtung (10) zur mechanischen Stabilisierung eines Anschlusses, insbesondere eines Sensoranschlusses, an einem flexiblen Beutel (30), mit
einem starren Stützteil (12), das aus wenigstens zwei Teilen (42) zusammensetzbar ist,
wobei die Teile (42) im zusammengesetzten Zustand zusammen einen freien Bereich oder eine Öffnung (16) umgeben, in die ein in den Beutel (30) integriertes starres Anschlussteil, insbesondere ein Sensor (14), platziert werden kann,
wobei der freie Bereich oder die Öffnung (16) im zusammengesetzten Zustand der Teile (42) einen kleineren Durchmesser aufweist als das Anschlussteil, sodass die Teile (42) eine Lastaufnahme für das Anschlussteil bilden.

2. Vorrichtung (10) nach Anspruch 1, **dadurch gekennzeichnet, dass** die Teile (42) im zusammengesetzten Zustand ein im Wesentlichen U-förmiges Stützteil (12) ergeben.

3. Vorrichtung (10) nach Anspruch 1 oder 2, **dadurch gekennzeichnet, dass** die Teile (42) mithilfe eines Verbindungselements (46) verbunden sind, wobei ein Abschnitt des Verbindungselements (46) von einem der Teile (42) vorsteht und in eine an einem anderen der Teile (42) vorgesehene, auf den vorstehenden Abschnitt des Verbindungselements (46) abgestimmte Öffnung eingeführt ist.

4. Vorrichtung (10) nach einem der vorhergehenden Ansprüche, **dadurch gekennzeichnet, dass** eine Unterseite der Teile (42) eine auf die Form des Anschlussteils abgestimmte Aufnahme (48) aufweist.

5. Vorrichtung (10) nach einem der vorhergehenden Ansprüche, **dadurch gekennzeichnet, dass** das Stützteil (12) an entgegengesetzten Enden abgeflachte Vorsprünge (44) aufweist.

6. Vorrichtung (10) nach einem der vorhergehenden Ansprüche, **dadurch gekennzeichnet, dass** das Stützteil (12) als Einweg-Komponenten ausgelegt ist und aus einem sterilisierbaren Kunststoff besteht.

7. Vorrichtungsanordnung, mit einem flexiblen Beutel (30), einem in eine Wand des Beutels (30) integrierten starren Anschlussteil und einer Stabilisierungsvorrichtung (10) nach einem der vorhergehenden Ansprüche.

8. Vorrichtungsanordnung nach Anspruch 7, **gekennzeichnet durch** einen starren Behälter (38), in dem der Beutel (30) aufgenommen ist und von dem der Beutel (30) abgestützt wird, wobei der starre Behälter (38) eine Fensteröffnung (40) hat, durch die das in den Beutel (30) integrierte Anschlussteil zugänglich ist.

9. Vorrichtungsanordnung nach Anspruch 8, **dadurch gekennzeichnet, dass** das Stützteil (12) eine Form aufweist, die an eine Kontur der Innenwand des Behälters (38) im Bereich der Fensteröffnung (40) angepasst ist.

10. Vorrichtungsanordnung nach Anspruch 8 oder 9, **dadurch gekennzeichnet, dass** die zwei Teile (42) Streben sind.

11. Vorrichtungsanordnung nach Anspruch 10, **dadurch gekennzeichnet, dass** der flexible Beutel (30) ein Einweg-Bioreaktor und das Anschlussteil ein Sensor (14) ist.

12. Vorrichtungsanordnung nach Anspruch 11, **dadurch gekennzeichnet, dass** die beiden Streben (42) einzeln zwischen einen Rand der Fensteröffnung (40) und die Außenwand des Bioreaktors geklemmt sind, wobei die Streben 42 auf entgegengesetzten Seiten des Sensors (14) positioniert sind.

13. Vorrichtungsanordnung nach einem der Ansprüche 8 bis 12, **dadurch gekennzeichnet, dass** der Beutel (30), das Anschlussteil und die Stabilisierungsvorrichtung (10) als sterilisierbare Einwegkomponenten ausgelegt sind.

## Claims

1. Device (10) for the mechanical stabilisation of a connection,
in particular a sensor connection, on a flexible bag (30), comprising a rigid support part (12) which is assemblable from at least two parts (42), wherein the parts (42) in the assembled state together surround a free area or an opening (16) in which a rigid connection part integrated in the bag (30), in particular a sensor (14), can be placed,
wherein the free area or the opening (16) in the assembled state of the parts (42) has a smaller diameter than the connection part, so that the parts (42) form a load bearing for the connection part.

2. Device (10) according to claim 1, **characterised in that** the parts (42) in the assembled state form an essentially U-shaped support part (12).

3. Device (10) according to claim 1 or 2, **characterised in that** the parts (42) are connected by means of a connecting element (46), wherein a section of the connecting element (46) protrudes from one of the parts (42) and is inserted into an opening adapted to the protruding section of the connecting element (46), provided on another of the parts (42).

4. Device (10) according to one of the preceding claims, **characterised in that** an underside of the parts (42) has a receptacle (48) adapted to the shape of the connection part.

5. Device (10) according to one of the preceding claims, **characterised in that** the support part (12) has flattened projections (44) at opposite ends.

6. Device (10) according to one of the preceding claims, **characterised in that** the support part (12) is designed as a single-use component and consists of a sterilisable plastics material.

7. Device arrangement, comprising a flexible bag (30), a rigid connection part integrated in a wall of the bag (30) and a stabilisation device (10) according to one of the preceding claims.

8. Device arrangement according to claim 7, **characterised by** a rigid container (38) in which the bag (30) is received and by which the bag (30) is supported, wherein the rigid container (38) has a window opening (40) through which the connection part integrated in the bag (30) is accessible.

9. Device arrangement according to claim 8, **characterised in that** the support part (12) has a shape which is adapted to a contour of the inner wall of the container (38) in the area of the window opening (40).

10. Device arrangement according to claim 8 or 9, **characterised in that** the two parts (42) are struts.

11. Device arrangement according to claim 10, **characterised in that** the flexible bag (30) is a single-use bioreactor and the connection part is a sensor (14).

12. Device arrangement according to claim 11, **characterised in that** the two struts (42) are individually clamped between an edge of the window opening (40) and the outer wall of the bioreactor, wherein the struts (42) are positioned on opposite sides of the sensor (14).

13. Device arrangement according to one of claims 8 to 12, **characterised in that** the bag (30), the connection part and the stabilisation device (10) are designed as sterilisable single-use components.

## Revendications

1. Dispositif (10) pour la stabilisation mécanique d'un raccord,
en particulier d'un raccord de capteur, sur une poche flexible (30), comportant un élément de support rigide (12) qui est assemblable à partir d'au moins deux parties (42),
dans lequel les parties (42) à l'état assemblé entourent ensemble une zone libre ou une ouverture (16) dans laquelle peut être placée une pièce de raccordement rigide intégrée dans la poche (30), en particulier un capteur (14),
dans lequel la zone libre ou l'ouverture (16) à l'état assemblé des parties (42) présente un diamètre plus petit que la pièce de raccordement, de sorte que les parties (42) forment une reprise de charge pour la pièce de raccordement.

2. Dispositif (10) selon la revendication 1, **caractérisé en ce que** les parties (42) à l'état assemblé forment un élément de support (12) essentiellement en forme de U.

3. Dispositif (10) selon la revendication 1 ou 2, **caractérisé en ce que** les parties (42) sont reliées au moyen d'un élément de liaison (46), dans lequel une section de l'élément de liaison (46) fait saillie d'une des parties (42) et est insérée dans une ouverture adaptée à la section saillante de l'élément de liaison (46), prévue sur une autre des parties (42).

4. Dispositif (10) selon l'une quelconque des revendications précédentes, **caractérisé en ce qu'**une face inférieure des parties (42) présente un logement (48) adapté à la forme de la pièce de raccordement.

5. Dispositif (10) selon l'une quelconque des revendications précédentes, **caractérisé en ce que** l'élément de support (12) présente à ses extrémités opposées des saillies aplaties (44).

6. Dispositif (10) selon l'une quelconque des revendications précédentes, **caractérisé en ce que** l'élément de support (12) est conçu en tant que composant à usage unique et est constitué d'un matériau synthétique stérilisable.

7. Agencement de dispositifs, comportant une poche flexible (30), une pièce de raccordement rigide intégrée dans une paroi de la poche (30) et un dispositif de stabilisation (10) selon l'une quelconque des revendications précédentes.

8. Agencement de dispositifs selon la revendication 7, **caractérisé par** un récipient rigide (38) dans lequel la poche (30) est reçue et par lequel la poche (30) est soutenue, dans lequel le récipient rigide (38) comporte une ouverture de fenêtre (40) à travers laquelle la pièce de raccordement intégrée dans la poche (30) est accessible.

9. Agencement de dispositifs selon la revendication 8, **caractérisé en ce que** l'élément de support (12) présente une forme qui est adaptée à un contour de la paroi intérieure du récipient (38) dans la zone de l'ouverture de fenêtre (40).

10. Agencement de dispositifs selon la revendication 8 ou 9, **caractérisé en ce que** les deux parties (42) sont des entretoises.

11. Agencement de dispositifs selon la revendication 10, **caractérisé en ce que** la poche flexible (30) est un bioréacteur à usage unique et la pièce de raccordement est un capteur (14).

12. Agencement de dispositifs selon la revendication 11, **caractérisé en ce que** les deux entretoises (42) sont serrées individuellement entre un bord de l'ouverture de fenêtre (40) et la paroi extérieure du bioréacteur, dans lequel les entretoises (42) sont positionnées sur des côtés opposés du capteur (14).

13. Agencement de dispositifs selon l'une quelconque des revendications 8 à 12, **caractérisé en ce que** la poche (30), la pièce de raccordement et le dispositif de stabilisation (10) sont conçus en tant que composants à usage unique stérilisables.
